# EUROPEAN PATENT APPLICATION

(11) **EP 4 567 016 A1**
(43) Date of publication of application: **11.06.2025**
(21) Application number: 24217426.6
(22) Date of filing: 04.12.2024
(51) Int. Cl.: C04B 35/486, C04B 35/488, C04B 35/626, C04B 35/645, A61K 6/78, A61K 6/818, A61C 13/08, A61K 6/58, A61L 2/08

(54) **ZIRCONIA-BASED IMPLANT COMPOSITION**

(30) Priority: 06.12.2023 KR 20230175679
(71) Applicant: Vatech mcis co., Ltd, Suwon-si, Gyeonggi-do (KR)
(72) Inventor: KIM, Dae Jun, Hwaseong-si (KR); YANG, Gi Uk, Hwaseong-si (KR); HONG, Ju Seop, Hwaseong-si (KR)
(74) Representative: Lorenz, Werner

(57) **Abstract**

Proposed is a zirconia-based implant composition capable of preventing discoloration under gamma irradiation. The zirconia-based implant composition is characterized by including zirconium dioxide (ZrO₂), yttrium oxide (Y₂O₃) added to the above zirconium dioxide, an oxide of pentavalent ions added for reducing the concentration of oxygen vacancies caused by the above yttrium oxide (Y₂O₃), a colorant oxide added for reducing discoloration, and an oxide of tetravalent ions added for improving sinterability.

## Description

### CROSS REFERENCE TO RELATED APPLICATION

The present application claims priority to Korean Patent Application No. 10-2023-0175679, filed December 6, 2023, the entire contents of which is incorporated herein for all purposes by this reference.

### BACKGROUND

### 1. Technical Field

The present disclosure relates to a dental implant composition and, particularly, to a zirconia-based implant composition capable of preventing discoloration even under gamma irradiation.

### 2. Description of the Related Art

Existing artificial teeth capable of replacing natural teeth were primarily made of metals, but ceramic prostheses being highly aesthetic and capable of showing colors similar to those of natural teeth have begun to be introduced. Recently, zirconia, a hypoallergenic material that is highly aesthetic due to showing colors similar to those of teeth, excellent in terms of strength, and human friendly, has become popular as a dental implant material.

Various sterilization methods, such as those using autoclave steam, H₂O₂ plasma, ethylene oxide gas, and gamma irradiation, can be applied to sterilize zirconia-based implants. The first three methods mentioned above are only effective in sterilizing surfaces with which sterilizing media come into contact. For this reason, there are difficulties in these processes since sterilization needs to be performed at each inner packaging step to sterilize implants while being packaged.

On the other hand, gamma irradiation allows for sterilization of implants while being packaged and thus is the most practical sterilization method. However, there are problems in that the excitation of oxygen vacancies, serving as color centers in zirconia, causes changes in the color of implants to black during gamma irradiation, resulting in a loss of aesthetics.

As solutions for restoring color changes caused by gamma rays, German Patent No. DE10201410 has disclosed a method of performing thermal treatment in a temperature range of 60 to 150 degrees, and Korean Patent No. 10-1640562 has disclosed a method of performing thermal treatment in a temperature range of 100 to 300 degrees. However, such thermal treatment causes deformation of packaging materials, which is problematic. Therefore, U.S. Patent Application Publication No. 2019/0001011 A1 has disclosed a method of restoring color by electromagnetic irradiation within visible rays in a wavelength range of 150 to 700 nm as a solution to these problems.

Once a new method capable of fundamentally preventing color changes of zirconia-based implants during gamma irradiation is developed, additional methods, such as thermal treatment or irradiation, for restoring the color changes caused by gamma rays will be unnecessary.

### [Document of related art]

### [Patent Document]

(Patent Document 1) Korean Patent No. 10-1640562
(Patent Document 2) U.S. Patent Application Publication No. 2019/0001011 A1

### SUMMARY

Accordingly, the present disclosure, which has been invented in response to the needs described above, primarily aims to provide a zirconia-based implant composition capable of preventing or minimizing implant discoloration caused by gamma irradiation.

Furthermore, the present disclosure aims to provide a zirconia-based implant composition capable of increasing the surface tension during a thermal treatment process to improve osseointegration properties during implant placement, thereby increasing the implant success rate.

In order to achieve the objectives described above, a zirconia-based implant composition, according to one embodiment of the present disclosure, is characterized by including zirconium dioxide (ZrO₂), yttrium oxide (Y₂O₃), an oxide of pentavalent ions, and at least one colorant oxide of erbium oxide (Er₂O₃), europium oxide (Eu₂O₃), and iron oxide (Fe₂O₃).

Furthermore, the zirconia-based implant composition described above is characterized in that the oxide of the pentavalent ions is niobium pentoxide (Nb₂O₅), an oxide of tetravalent ions is further included, and the oxide of the tetravalent ions includes at least one of silicon dioxide (SiO₂), titanium dioxide (TiO₂), and cerium oxide (CeO₂).

In addition, the zirconia-based implant composition described above is characterized in that an oxide of tetravalent ions is further included, the oxide of the pentavalent ions is niobium pentoxide (Nb₂O₅), the oxide of the tetravalent ions includes silicon dioxide (SiO₂), titanium dioxide (TiO₂), and cerium oxide (CeO₂), zirconium dioxide (ZrO₂) has a content in the range of 79.0 to 93.6 mol%, yttrium oxide (Y₂O₃) has a content in the range of 3.0 to 6.0 mol%, the oxide of the pentavalent ions has a content in the range of 3.0 to 10.0 mol%, the colorant oxide has a content in the range of 0.2 to 2.0 mol%, silicon dioxide (SiO₂) has a content in the range of 0.01 to 1.0 mol%, titanium dioxide (TiO₂) has a content in the range of 0.01 to 1.0 mol%, and cerium oxide (CeO₂) has a content in the range of 0.2 to 2.0 mol%.

According to the means of solving the technical problems described above, the present disclosure can cause compositional changes in a zirconia-based implant material to reduce the concentration of oxygen vacancies responsible for discoloration while adding colorant oxides for reducing discoloration to black, thereby minimizing discoloration caused by gamma irradiation.

Furthermore, in this process, the surface tension can be increased through an additional thermal treatment process to improve osseointegration properties during implant placement, thereby providing the effect of increasing the implant success rate.

### BRIEF DESCRIPTION OF THE DRAWINGS

FIG. 1 is an exemplary diagram showing changes in color of a zirconia-based implant sample, according to one embodiment of the present disclosure, before/after gamma irradiation;
FIG. 2 is a diagram showing changes in contact angle and surface free energy of a zirconia-based implant sample, according to one embodiment of the present disclosure, before/after gamma irradiation and during thermal treatment; and
FIG. 3 is a graph showing changes in surface free energy of a zirconia-based implant sample, according to one embodiment of the present disclosure, as a function of thermal treatment temperature after gamma irradiation.

### DETAILED DESCRIPTION

The present disclosure will be described in detail below with reference to the accompanying drawings illustrated as examples of specific embodiments under which the present disclosure may be implemented to make clear of the objectives, technical solutions, and advantages of the present disclosure. These embodiments are described in sufficient detail so that those skilled in the art can readily practice the present disclosure.

Furthermore, throughout the detailed description and claims of the present disclosure, the terms, such as "include" and "comprise", and variations thereof are not intended to exclude other technical features, additions, components, or steps. Other objectives, advantages, and features of the present disclosure will be revealed to those skilled in the art, partially from this specification and partially from the implementation of the present disclosure. The following examples and drawings are provided as examples and are not intended to limit the present disclosure. Moreover, the present disclosure encompasses all possible combinations of embodiments indicated herein. It should be understood that various embodiments of the present disclosure are different but are not necessarily mutually exclusive. The following detailed description is, therefore, not to be taken in a limiting sense, and the scope of the present disclosure is defined only by the appended claims, when appropriately interpreted, along with the full range of equivalents to what the claims claim.

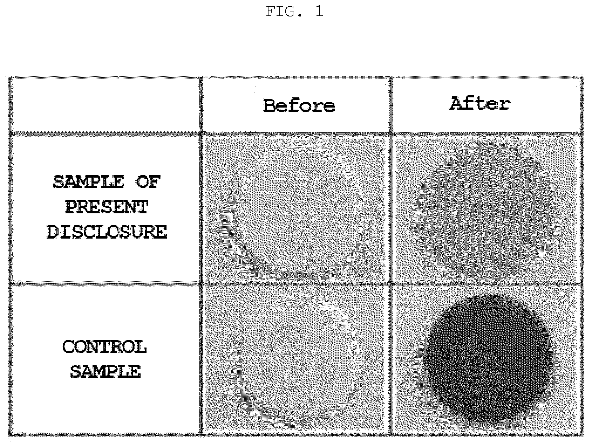

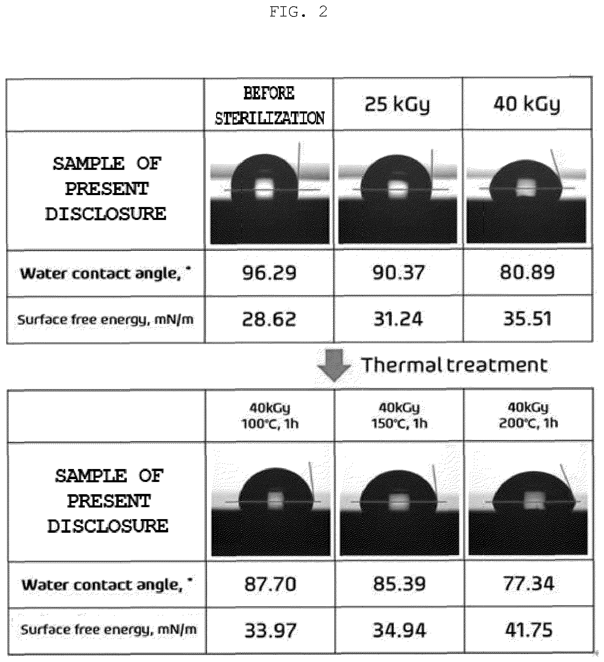

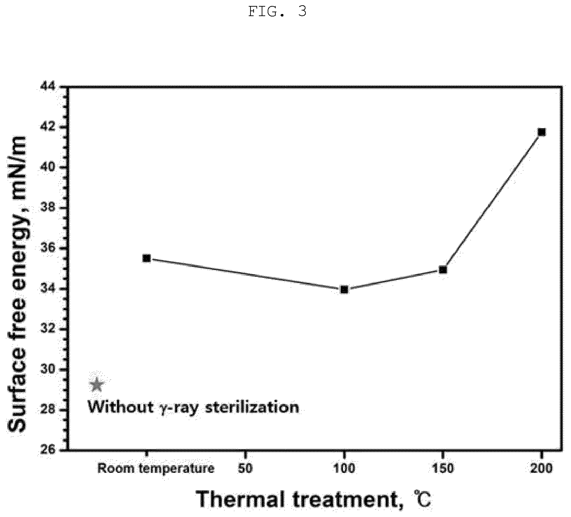

FIG. 1 illustrates changes in color of a zirconia-based implant sample, according to one embodiment of the present disclosure, before/after gamma irradiation, FIG. 2 shows changes in contact angle and surface free energy of a zirconia-based implant sample, according to one embodiment of the present disclosure, before/after gamma irradiation and during thermal treatment, and FIG. 3 is a graph showing changes in surface free energy of a zirconia-based implant sample, according to one embodiment of the present disclosure, as a function of thermal treatment temperature after gamma irradiation.

Typically, compositional changes in zirconia are problematic in terms of deterioration in mechanical properties or increased vulnerability to low-temperature degradation. In one embodiment of the present disclosure, additives that address discoloration issues in zirconia-based implants sterilized by gamma irradiation will be presented to describe examples regarding discoloration prevention and changes in mechanical properties.

First of all, these additives may reduce the concentration of oxygen vacancies caused by Y ions with an oxidation state of 3 that are fundamentally added to zirconia, the implant material, by adding oxides of pentavalent ions. To this, colorant oxides for reducing discoloration to black may be added, as shown in FIG. 1, to minimize discoloration caused by gamma irradiation. Furthermore, in this process, the surface tension may be increased during an additional thermal treatment process to improve osseointegration properties during implant placement, thereby increasing the implant success rate.

For the detailed description thereof, a zirconia-based implant composition, according to one embodiment of the present disclosure, includes zirconium dioxide (ZrO₂), yttrium oxide (Y₂O₃), an oxide of pentavalent ions added for reducing the concentration of oxygen vacancies caused by the above yttrium oxide (Y₂O₃), a colorant oxide added for reducing discoloration, and an oxide of tetravalent ions added for improving sinterability.

The oxide of the pentavalent ions may be niobium pentoxide (Nb₂O₅), and the colorant oxide may be any one of erbium oxide (Er₂O₃), europium oxide (Eu₂O₃), and iron oxide (Fe₂O₃).

The oxide of the tetravalent ions includes silicon dioxide (SiO₂), titanium dioxide (TiO₂), and cerium oxide (CeO₂). Silicon dioxide (SiO₂) and titanium dioxide (TiO₂) are additives that help improve sinterability, while cerium oxide (CeO₂) serves as a colorant oxide.

The composition, types of oxides, and content ranges thereof for the zirconia-based implant composition, according to one embodiment of the present disclosure, are as follows.

Zirconium dioxide (ZrO₂) has a content in the range of 79.0 to 93.6 mol%, yttrium oxide (Y₂O₃), which is an oxide of trivalent ions and serves as a zirconia stabilizer, has a content in the range of 3.0 to 6.0 mol%, niobium pentoxide (Nb₂O₅), which is the oxide of the pentavalent ions, has a content in the range of 3.0 to 10.0 mol%, any one of erbium oxide (Er₂O₃), europium oxide (Eu₂O₃), and iron oxide (Fe₂O₃), serving as the colorant oxide for realizing the color of the gingiva, has a content in the range of 0.2 to 2.0 mol%, silicon dioxide (SiO₂) and titanium dioxide (TiO₂), which are the oxides of the tetravalent ions, each independently have a content in the range of 0.01 to 1.0 mol%, and cerium oxide (CeO₂) has a content in the range of 0.2 to 2.0 mol%.

### [Example]

Hereinafter, a zirconia-based implant sample, according to one example of the present disclosure (when referred to as a sample of the present disclosure), and a control sample (3Y-TZP, Tosoh cop) will be compared in terms of composition. The composition of the zirconia-based implant sample, according to one example of the present disclosure, and the composition of the control sample are as shown in Table 1 below.

**[Table 1]**

| | | | | | | | |
|---|---|---|---|---|---|---|---|
| Sample of present disclosure | ZrO₂ | Y₂O₃ | Nb₂O₅ | SiO₂ | TiO₂ | CeO₂ | Er₂O₃ |
| Molar concentration | 91.19 | 4.41 | 3.28 | 0.18 | 0.27 | 0.46 | 0.21 |
| Control sample | ZrO₂ | Y₂O₃ | HfO₂ | Al₂O₃ | SiO₂ | Fe₂O₃ | Na₂O |
| Molar concentration | 93.64 | 3 | 3.1 | 0.13 | 0.04 | 0.01 | 0.08 |

First of all, the changes in the colors of the sample of the present disclosure and the control sample before and after gamma irradiation are as shown in FIG. 1. As shown in Table 1, the zirconia-based implant sample, according to one example of the present disclosure, reduces the concentration of oxygen vacancies caused by yttrium oxide (Y₂O₃), which is the oxide of the trivalent ions fundamentally added to zirconia, by adding niobium pentoxide (Nb₂O₅), which is the oxide of the pentavalent ions. In addition, compared to the case of the control sample (3Y-TZP) in FIG. 1, the effect of minimizing the discoloration of the sample of the present disclosure to black caused by gamma irradiation can be obtained by adding the colorant oxide.

Referring to FIG. 2, in which the water contact angle and surface free energy of the sample of the present disclosure are measured before and after gamma irradiation and during thermal treatment, the contact angle between γ _{SL} and γ _{LG} appears to decrease before gamma irradiation (before sterilization) and after gamma irradiation, while the surface free energy relatively appears to increase.

In addition, when performing an additional thermal treatment process on the sample irradiated with gamma rays, the surface free energy appears to increase, indicating that during the additional thermal treatment process, the surface tension is increased, and osseointegration properties can thus be improved during implant placement.

Referring to FIG. 3, which is a graph showing changes in the surface free energy as a function of thermal treatment temperature after gamma irradiation, the surface free energy appears to dramatically increase in a range where the temperature of a room is 150°C or higher.

According to the example described above, the present disclosure can cause compositional changes in the zirconia-based implant material to reduce the concentration of oxygen vacancies responsible for discoloration while adding the colorant oxides for reducing discoloration to black, thereby minimizing discoloration caused by gamma irradiation. Furthermore, in this process, the surface tension can be increased through the additional thermal treatment process to improve osseointegration properties during implant placement, thereby obtaining the effect of increasing the implant success rate.

For reference, a manufacturing process of the implant having the composition ratios as listed in Table 1 is to be further described.

First, zirconium dioxide (ZrO₂), yttrium oxide (Y₂O₃), and niobium pentoxide (Nb₂O₅), serving as the raw materials, are prepared, mixed, and subjected to solution treatment, followed by adding silicon dioxide (SiO₂) and titanium dioxide (TiO₂), serving as sintering agents, and cerium oxide (CeO₂) and erbium oxide (Er₂O₃), serving as colorant oxides. Then, an injection molding process is performed to shape an implant.

Subsequently, degreasing and sintering processes are performed to remove organic matter and impart sintering properties, and hot isostatic pressing (HIP) and thermal treatment processes are then performed for densification and color restoration. Next, gamma irradiation is performed for sterilization, and an additional thermal treatment process is performed for color restoration.

Although the present disclosure has been described hereinabove with reference to the embodiment shown in the drawing, this is merely exemplary, and those skilled in the art will understand that various modifications and other equivalent embodiments are possible therefrom. For example, even though dental implants were illustrated in the embodiment of the present disclosure, the embodiment of the present disclosure can be equally applied to surgical implants. Thus, the true technical protection scope of the present disclosure should be defined by the technical ideas of the appended claims.

## Claims

1. A zirconia-based implant composition comprising:
zirconium dioxide (ZrO₂);
yttrium oxide (Y₂O₃) ;
an oxide of pentavalent ions; and
at least one colorant oxide of erbium oxide (Er₂O₃), europium oxide (Eu₂O₃), and iron oxide (Fe₂O₃).

2. The implant composition of claim 1, wherein the oxide of the pentavalent ions is niobium pentoxide (Nb₂O₅).

3. The implant composition of claim 1, further comprising an oxide of tetravalent ions.

4. The implant composition of claim 3, wherein the oxide of the tetravalent ions comprises at least one of silicon dioxide (SiO₂), titanium dioxide (TiO₂), and cerium oxide (CeO₂).

5. The implant composition of claim 1, further comprising an oxide of tetravalent ions,
wherein the oxide of the pentavalent ions is niobium pentoxide (Nb₂O₅),
the oxide of the tetravalent ions comprises silicon dioxide (SiO₂), titanium dioxide (TiO₂), and cerium oxide (CeO₂),
zirconium dioxide (ZrO₂) has a content in a range of 79.0 to 93.6 mol%,
yttrium oxide (Y₂O₃) has a content in a range of 3.0 to 6.0 mol%,
the oxide of the pentavalent ions has a content in a range of 3.0 to 10.0 mol%,
the colorant oxide has a content in a range of 0.2 to 2.0 mol%, silicon dioxide (SiO₂) has a content in a range of 0.01 to 1.0 mol%,
titanium dioxide (TiO₂) has a content in a range of 0.01 to 1.0 mol%, and
cerium oxide (CeO₂) has a content in a range of 0.2 to 2.0 mol%.
